# EUROPEAN PATENT APPLICATION

(11) **EP 4 289 287 A1**
(43) Date of publication of application: **13.12.2023**
(21) Application number: 22876777.8
(22) Date of filing: 27.09.2022
(51) Int. Cl.: A23L 33/10, A23P 10/47, A23L 3/46, A23L 3/44, A61K 9/127

(54) **CURCUMIN NANOPARTICLE COMPLEX HAVING MODIFIED PARTICLE SURFACE AND METHOD FOR PREPARING SAME**

(30) Priority: 28.09.2021 KR 20210127770; 24.12.2021 KR 20210187174
(71) Applicant: Binotec Co., Ltd., Daegu 42151 (KR)
(72) Inventor: JANG, Gi Hyun, Daegu 42151 (KR); SEOK, Chang Hwan, Daegu 42151 (KR); SHIN, Ji Won, Daegu 42151 (KR); GWAK, Yun Geum Sang, Daegu 42151 (KR); KIM, Yu Mi, Daegu 42093 (KR)
(74) Representative: Mewburn Ellis LLP
(86) International application number: PCT/KR2022/014386
(87) International publication number: WO 2023/055005

(57) **Abstract**

Provided are a curcumin nanoparticle complex having a modified particle surface and a method for preparing same. A curcumin nanoparticle complex includes curcumin and phospholipids and has a particle size of less than 1000 nm. A first coating layer containing a cationic material and/or a second coating layer containing an anionic material can be further added.

## Description

### Technical field

The present invention relates to a curcumin nanoparticle complex having a modified particle surface and a method for preparing the same.

### Background art

The public interest in immune-boosting which has antiviral and anti-inflammatory functions has rapidly increased worldwide due to COVID-19. Accordingly, the interest in health functional food is increasingly growing, and the interest in curcumin which has broad efficacy and particularly has excellent anti-inflammatory, antiviral, antibacterial and anticancer effects is greatly on the rise. Curcumin, which is a polyphenol found in turmeric (Curcuma longa) produced in India, is a safe food ingredient that does not cause side effects even with a dose up to 8 g/day in adults. Such curcumin exhibits excellent efficacy in *in vitro* experiments and many studies on the mechanism thereof have been carried out. However, when applied to human bodies, curcumin has very low bioavailability, and thus substantial efficacy can hardly be expected. This is because of the following properties of curcumin: low water solubility, instability in an aqueous solution phase and fast metabolism *in vivo.*

In order to overcome the above problems, many studies have been carried out to improve bioavailability (Grynkiewicz G, Slifirski P, Acta Biochim Pol 2012;59(2):201-12). If piperine, a component of black pepper, is contained to increase bioavailability of curcumin, liver metabolism of curcumin is disrupted, resulting in an increase in blood curcumin concentration. Also, many study results show that curcumin is formulated in nano liposomes, micelles, solid lipid nanoparticles, etc., to increase water solubility and absorption, thereby increasing its bioavailability. Products commercialized based thereon are Meriva, LongVida, CurQfen, NovaSol, Theracumin, etc. These products increase absorption of curcumin and exhibit over several-fold to hundred-fold higher concentration in the blood (Rohitash. J., Journal of Integrative Medicine, 12(2018), 367-374).

Particularly, Meriva contains phytosome, a complex of curcumin and phospholipids. WO2007/101551 discloses that curcumin-phospholipids complexes have been developed by mixing curcumin dissolved in ethanol with phospholipids such that the enolic hydroxyl group of curcumin and the polar section of phospholipids form a hydrogen bond as a complex, and evaporating ethanol and drying. However, the curcumin-phospholipids complexes were prepared by simply evaporating a solvent and drying, and thus no uniform nanoparticles can be formed. The uniformity of particles and the size of nanoparticles greatly affect improvement of blood concentration. As such, a technology that can form nanoparticles uniformly and enable mass production has not been fully completed yet over the world.

Phospholipids are a representative surfactant including a nonpolar saturated fatty acid and a polar phosphate group. Phospholipids are named lecithin when 80% or less of phospholipids are contained. Phospholipids include phosphatidylcholine, phosphatidylethanolamine, phosphatidylinocitol, etc. When specific phospholipids, phosphatidylcholine, are contained in an amount of 90% or more, the phospholipids may be named phosphatidylcholine. Phospholipids are very safe such that no intake limit therefore is given, and people of all ages and both sexes can take phospholipids because the substance is extracted from natural sources such as egg yolk, soybeans, sunflower seeds, etc. Furthermore, phospholipids have efficacy *per se* such as preventing arteriosclerosis, etc.

Phospholipids, which are similar to cell membranes, form two layers in an aqueous medium, allowing a water soluble substance to be encapsulated thereinside and a fat soluble substance to be encapsulated between the two layers of the phospholipids. This formulation is called a liposome, which is the representative formulation using a surfactant of phospholipids.

Curcumin is too poorly water soluble to be encapsulated in the form of liposomes, and thus it is impossible for the substance to be encapsulated in phospholipids and be present in a colloidal state. Accordingly, curcumin is mostly developed into solid lipid nanoparticles such as stearic acid or into micelles, or prepared and used in curcumin nanoparticles by dissolving curcumin in a solvent and then precipitating the same again (Dutta AK, et.al, J Bioequiv Availab 2013,6:1-9, Yallapu MM, et al Drug Disov Today 17:71-80, Donsi et al, J Agric Food Chem 2010 58:2848-2853).

It is difficult to powder solid lipid nanoparticles or micelles for commercialization. In the solid lipid nanoparticles or micelles, a large amount of surfactant is to be added. For example, in the case of a commercialized curcumin NovaSol, 93% of tween80 is contained. Such formulations can hardly be processed into a powdered form and thus are prepared in a liquid form. However, such a liquid form has many limitations from application fields to shelf life.

In addition, in order to increase absorption in the small intestine, most of all, a substance is to be adhered to the small intestine. The mucous membrane in the small intestine has bicarbonate (HCO3-) anions to neutralize stomach acid. Because of the anionic properties of the mucous membrane in the small intestine, particles with a cationic charge are more likely to be absorbed in epithelial cells in the small intestine. Therefore, it is required to develop particles with a cationic charge.

There is a need to develop a technology of crystallizing curcumin into uniform nanoparticles using phospholipids, a natural surfactant, to facilitate aqueous dispersion, modifying the surface with a cationic charge to be adhered to the intestine, and powdering into fine particles in a constant form to increase reproductivity.

### Summary of invention

### Technical task

The present invention is to provide a curcumin nanoparticle complex comprising curcumin and phospholipids (e.g., lecithin) and having a particle size of less than 1000 nm, wherein the curcumin nanoparticle complex can further comprise a first coating layer comprising a cationic material (e.g., chitosan) and/or a second coating layer comprising an anionic material (e.g., sodium alginate).

The present invention is to provide a composition comprising the curcumin nanoparticle complex.

The present invention is to provide a method for preparing the curcumin nanoparticle complex.

### Means for solving technical task

The present invention provides a curcumin nanoparticle complex comprising curcumin and phospholipids and having a particle size of less than 1000 nm, wherein the curcumin nanoparticle complex can further comprise a first coating layer comprising a cationic material and/or a second coating layer comprising an anionic material.

In an aspect, the present invention provides a curcumin nanoparticle complex comprising: curcumin; and about 6 parts by weight or more, e.g., about 8 parts by weight or more, specifically about 8 to 20 parts by weight of phospholipids, e.g., lecithin, relative to 1 part by weight of curcumin, wherein the curcumin nanoparticle complex can further comprise a first coating layer comprising about 0 to 0.3 parts by weight, specifically about more than 0 and 0.2 or less parts by weight, more specifically about 0.03 to 0.2 parts by weight of a cationic material, e.g., chitosan, relative to 1 part by weight of curcumin; and/or a second coating layer comprising about 0 to 0.025 parts by weight, specifically about more than 0 and 0.02 or less parts by weight, more specifically about 0.0001 to 0.02 parts by weight or about 0.0002 to 0.02 parts by weight, most specifically about 0.0003 to 0.02 parts by weight of an anionic material, e.g., sodium alginate, relative to 1 part by weight of curcumin, wherein the second coating layer is comprised only when the first coating layer is comprised.

In a preferred aspect, the curcumin nanoparticle complex comprises a first coating layer. In this case, the first coating layer is comprised in an amount of more than 0 parts by weight.

Accordingly, the present invention provides a curcumin nanoparticle complex comprising: curcumin; about 6 parts by weight or more, e.g., about 8 parts by weight or more, specifically about 8 to 20 parts by weight of phospholipids, e.g., lecithin, relative to 1 part by weight of curcumin; and a first coating layer comprising about more than 0 and 0.2 or less parts by weight, specifically about 0.03 to 0.2 parts by weight of a cationic material, e.g., chitosan, relative to 1 part by weight of curcumin.

In another aspect, the curcumin nanoparticle complex further comprises a second coating layer comprising about more than 0 and 0.02 or less parts by weight, more specifically about 0.0001 to 0.02 parts by weight or about 0.0002 to 0.02 parts by weight, most specifically about 0.0003 to 0.02 parts by weight of an anionic material, e.g., sodium alginate.

In a specific aspect, the present invention provides a curcumin nanoparticle complex comprising: curcumin; and about 8 to 20 parts by weight, e.g., about 8 to 20 parts by weight of lecithin, relative to 1 part by weight of curcumin.

In another specific aspect, the present invention provides a curcumin nanoparticle complex comprising: curcumin; 8 parts by weight of lecithin relative to 1 part by weight of curcumin; and a first coating layer comprising 0.03 parts by weight of chitosan relative to 1 part by weight of curcumin.

In another specific aspect, the present invention provides a curcumin nanoparticle complex comprising: curcumin; 8 parts by weight of lecithin relative to 1 part by weight of curcumin; a first coating layer comprising 0.03 parts by weight of chitosan relative to 1 part by weight of curcumin; and a second coating layer comprising 0.0003 parts by weight of sodium alginate relative to 1 part by weight of curcumin.

In an embodiment, when the content of phospholipids, e.g., lecithin, is less than the above numerical value, the amount of lecithin is insufficient to encapsulate curcumin, resulting in deposition of curcumin, and thus the encapsulation efficiency of curcumin may be decreased. When the content thereof is more than the above numerical value, the amount of lecithin is sufficient to encapsulate curcumin, but the uniformity of particles may be reduced due to unreacted lecithin.

In another embodiment, when the content of the cationic or anionic material is more than the above numerical value, deposition and layer separation may occur, resulting in a decrease in stability.

However, even beyond the numerical values, if "nanoparticle complexes" can be formed while maintaining the stability thereof, they can fall within the scope of the present application. This can be easily confirmed by a person skilled in the art through methods commonly used in the art.

The term "nanoparticle," as used herein, refers to particles in which curcumin is crystallized in uniform nanoparticles using phospholipids, with the size of nm. That is, the nanoparticle has the size of less than 1000 nm, and is also referred to as "nanocrystal."

In an embodiment, the nanoparticle complex may have the size of less than 1000 nm, 900 nm or less, 800 nm or less, 700 nm or less, 600 nm or less, 500 nm or less, and specifically, 50 to 900 nm, 100 to 700 nm, 200 to 600 nm, and 300 to 400 nm.

When the nanoparticle complex has the size of less than the above numerical value, a drug may not be properly encapsulated. When it has the size of more than the above numerical value, the complex is not dispersed in a solution, leading to deposition and reduction of stability of particles, and the nanoparticle complex may not satisfy the definition of nanoparticle.

The term "phospholipids," as used herein, include naturally occurring, semisynthetic or synthetically prepared products, which can be used alone or in a mixture.

Examples of naturally occurring phospholipids are natural lecithin (phosphatidylcholine (PC) derivatives), e.g., lecithin obtainable from soy bean, sunflower or other plant or animal sources, preferably plant sources.

Examples of semisynthetic phospholipids are partially or fully hydrogenated derivatives of naturally occurring lecithin. For example, the phospholipids include phosphatidylcholine, ehtyl phosphatidylcholine, phosphatidylglycerol, phosphatidic acid, phosphatidylethanolamine, phosphatidylserine, phosphatidylinositol or fatty acid diester of sphingomyelin, but are not limited thereto.

The term "first coating layer," as used herein, refers to a first coating layer formed on the surface of a complex of curcumin and phospholipids, which may include a cationic material.

The term "cationic material," as used herein, refers to a material charged with a cationic material in an amount sufficient to provide a cationic charge to a curcumin nanoparticle complex. In an example, the positively charged material may include naturally occurring or synthetic cationic polymers.

The naturally occurring cationic polymer includes at least one positively charge modified derivative selected from the group consisting of guar gum, cellulose, protein, polypeptide, chitosan, lanolin and starch, but is not limited thereto.

The synthetic cationic polymer includes at least one positively charge modified derivative selected from the group consisting of Polyquatemium-4, Polyquatemium-5, Polyquatemium-6, Polyquatemium-10, Polyquaternium-39, Polyquaternium-44, Polyquaternium-46, distearyldimonium chloride, cinnamidopropyltrimonium chloride, cetrimonium chloride and guar hydroxypropyltrimonium chloride, but is not limited thereto.

Accordingly, the "cationic material" of the present invention may be at least one naturally occurring cationic polymer selected from the group consisting of guar gum, cellulose, protein, polypeptide, chitosan, lanolin and starch; or at least one synthetic cationic polymer selected from the group consisting of Polyquatemium-4, Polyquatemium-5, Polyquatemium-6, Polyquatemium-10, Polyquaternium-39, Polyquaternium-44, Polyquaternium-46, distearyldimonium chloride, cinnamidopropyltrimonium chloride, cetrimonium chloride and guar hydroxypropyltrimonium chloride. The polymer may be dissolved or dispersed in water or an organic solvent to be used.

In a specific aspect, the cationic material of the present invention is chitosan.

The term "second coating layer," as used herein, refers to a second coating layer formed on the first coating layer, which may include an anionic material. Accordingly, the curcumin nanoparticle complex of the present invention may comprise the first coating layer alone, or the first coating layer and the second coating layer simultaneously.

The term "anionic material," as used herein, may be an anionic hydrogel. Examples of monomers of the anionic hydrogel include a polymerizable acid such as acrylic acid, methacrylic acid, vinylsulfonic acid, vinyl phosphonic acid, maleic acid (including anhydrides thereof), fumaric acid, itaconic acid, 2-acrylamido-2-methylpropanesulfonic acid, alginic acid, and 2-acrylamido-2-methylpropanephosphonic acid, and a salt thereof, e.g., sodium salt, potassium salt or ammonium salt, an amide thereof, hydroxyalkyl ester, and amino or ammonium functional ester and amide.

In a specific aspect, the anionic material of the present invention may be sodium alginate or alginate hydrogel.

In another aspect, the present invention provides a microfine powder powdered from a curcumin nanoparticle complex. Specifically, the present invention provides a microfine powder powdered from a curcumin nanoparticle complex, comprising: the curcumin nanoparticle complex; and at least one water soluble polymer selected from the group consisting of maltodextrin, dextrin and starch. The nanoparticle of the present invention is present in nm units, e.g., less than 1000 nm, in a solution state, but the nanoparticle has a size of micrometer or more by fusing with a water soluble polymer when undergoing drying such as powder drying in the presence of the water soluble polymer.

The water soluble polymer may be comprised in an amount of about 20 to 30 parts by weight, e.g., about 26.67 parts by weight, relative to 1 part by weight of curcumin.

In another aspect, the present invention provides a composition comprising the curcumin nanoparticle complex or the powdered microfine powder.

The term "composition," as used herein, includes a pharmaceutical composition for being used as pharmaceuticals for humans, a veterinary composition for being used as pharmaceuticals for animals, or a diet product or food for humans or animals (e.g., functional food composition, i.e., food, beverage, feed, or pet food, or food, beverage, feed or pet food supplements), etc. Accordingly, the "composition," as used herein, includes all of the "pharmaceutical composition," "veterinary composition," "feed composition for animal," or "food composition."

Accordingly, the composition of the present invention comprises the curcumin nanoparticle complex or the powdered microfine powder; and a pharmaceutically acceptable excipient, a veterinary acceptable excipient or a food acceptable excipient, according to the use.

The term "pharmaceutically acceptable," "veterinary acceptable" or "food acceptable," as used herein, refers to a compound, substance, composition, carrier and/or dosage form, which is suitable for use in contact with tissues of humans or animals without causing excessive toxicity, stimulus, allergic reaction, or other symptoms or complications, within the sound determination of medicine or food, which meets the rational interest/risk ratio.

The term "pharmaceutically acceptable excipient," "veterinary acceptable excipient" or "food acceptable excipient," as used herein, refers to an excipient useful for preparing a pharmaceutical composition which is generally safe, nontoxic, and desirable biologically or in other ways, and includes an excipient acceptable for human pharmaceuticals, animal veterinary medicines, and food. The term "pharmaceutically acceptable excipient," "veterinary acceptable excipient" or "food acceptable excipient," as used in the specification and claims, includes one type or more of these excipients. For example, the pharmaceutically, veterinary, food acceptable excipient used in the preparation of the present invention may be a diluent or inert carrier, lubricant, binder or a combination thereof. The excipient used in the preparation of the present invention may further comprise a filler, anti-microbial agent, antioxidant, anticaking agent, coating agent or a mixture thereof. Other excipients that are pharmaceutically, veterinary or food acceptable can be used without any limitations.

The composition according to the present invention may be administered to humans or other mammals by various methods. Here, other mammals may be livestock or pet, such as dogs, cats, rabbits, pigs, sheep, goats, milk cows, horses, cows, etc., but are not limited thereto.

When the composition is a pharmaceutical composition, the pharmaceutical composition may be prepared for parenteral or oral administration. The representative dosage form of parenteral administration is an injectable dosage form, and preferably the composition may be an isotonic solution or suspension. The injectable dosage form may be prepared using suitable dispersants or humectants, suspending agents by methods known in the art. For example, each ingredient may be dissolved in a saline solution or buffer solution to be formulated into an injection. The oral dosage form includes powders, granules, tablets, pills, capsules, etc., but is not limited thereto.

The composition according to the embodiments may be prepared in various preparations using a drug carrier. The drug delivery system may be liposomes, ethosomes, elastic ethosomes, targeting liposomes, microparticles, microcapsules, nanoparticles, nanocapsules, nanofibers, etc., but is not limited thereto.

When the composition is a food composition, the composition may be used as, for example, a main ingredient or supplementary ingredient of food, food additives, functional food, beverage, etc. The food composition may be used as, for example, health functional food, vitamin complex, gum, beverage, various foods, tea, various meat products, fish products, tofus, jellies, noodles, bread, health supplements, seasonings, sauces, snacks, candies, dairy products, other processed food, fermented food, natural seasonings, etc., but is not limited thereto.

In another aspect, the present invention provides a method for preparing the curcumin nanoparticle complex.

In a specific aspect, the present invention provides a method for preparing the curcumin nanoparticle complex, comprising: (a) dissolving curcumin in an organic solvent to prepare a curcumin solution; (b) dissolving phospholipids, e.g., lecithin, in an organic solvent, water or a mixed solvent of the two to prepare a lecithin solution or dispersion; (c) mixing the curcumin solution of step (a) with the lecithin solution or dispersion of step (b) to prepare a curcumin-lecithin mixed solution; (d) mixing the curcumin-lecithin mixed solution with water to form an emulsion; and (e) high-pressure homogenizing the emulsion to prepare a curcumin nanoparticle complex, and optionally comprising: (f) performing first coating on the curcumin nanoparticle complex with a first coating agent comprising a cationic material, e.g., chitosan; and/or (g) performing second coating on the first coated curcumin nanoparticle complex with a second coating agent comprising an anionic material, e.g., sodium alginate. The content of each ingredient used is the same as described above.

In another aspect, the present invention provides a method for preparing a microfine powder powdered from a curcumin nanoparticle complex. Specifically, the present invention provides a method for preparing a microfine particle, comprising: preparing a curcumin nanoparticle complex by the aforementioned method; and further comprising: (g) adding at least one water soluble polymer selected from the group consisting of maltodextrin, dextrin and starch; and (h) performing powdering through spray drying or freeze drying. The content of each ingredient used is the same as described above.

In an embodiment, in step (f), the first coating agent comprising the cationic material may be a chitosan solution having a concentration of 1.5%(w/w) or more.

In another embodiment, in step (f), the second coating agent comprising the anionic material may be a sodium alginate solution having a concentration of 0.015%(w/w) or more.

### Effect of invention

The curcumin nanoparticle complex of the present invention has uniform nanocrystals, and a liposome encapsulating the uniform nanocrystals has a surface modified with a cationic material and/or an anionic material, thereby increasing absorption into the mucous membrane of the small intestine.

Also, the curcumin nanoparticle complex of the present invention forms a complex of a micro size through powdering, which achieves a sustained release effect, thereby increasing blood concentration and improving bioavailability.

Accordingly, the curcumin nanoparticle complex of the present invention exhibits effective anti-inflammatory, antiviral, antibacterial or anticancer activity, and thus can be widely used for pharmaceuticals, food or animal feed.

### Brief description of drawings

Fig. 1 shows the result of observing formulation stability immediately after preparing curcumin nanoparticle complexes of Example 1, Comparative Example 1 and Comparative Example 5;
Fig. 2 shows field emission scanning electron microscopy (FE-SEM) images of Comparative Example 1, Example 1 and Example 6;
Fig. 3 is a graph showing LPS-induced nitric oxide (NO) inhibition of the curcumin nanoparticle complex of the present invention (Experimental Example 4);
Fig. 4 is a graph showing a decrease in TNF-α expression of the curcumin nanoparticle complex of the present invention (Experimental Example 5);
Fig. 5 is a graph showing T cell (MOLT-4) proliferation of the curcumin nanoparticle complex of the present invention (Experimental Example 6);
Fig. 6 is a graph showing the result of *in vitro* release testing over time under artificial gastro fluid and artificial intestinal fluid conditions of the curcumin nanoparticle complex of the present invention (Experimental Example 7); and
Fig. 7 is a graph showing the curcumin concentration in the blood over time through *in vivo* animal testing after orally administering the curcumin nanoparticle complex of the present invention to SD rats (Experimental Example 8).

### Best mode for carrying out the invention

The embodiments of the present invention will be described in detail to an extent to be easily carried out by a person having ordinary knowledge in the art to which the present invention pertains with reference to the accompanying drawings. The present invention may be implemented into various modifications and is not limited to the embodiments described herein.

Hereinafter, the present invention is described in more detail with examples. However, the present invention is not limited to the following examples.

### Example: Preparation of Coated Curcumin Nanoparticle

Each ingredient was added according to the composition of Table 1, to prepare coated curcumin nanoparticles.

### Example 1

0.15 % by weight of curcumin powdered extract (Curcumin C3 Complex, Sabinsa) was added to ethanol and dissolved under 70°C temperature conditions, to prepare a curcumin solution (A). Separately, 1.2 % by weight of powdered lecithin (Emulpur IP, Cargill) was added to a solution in which ethanol and purified water are mixed, and then dispersed under 70°C temperature conditions, to prepare a lecithin dispersion (B).

The prepared curcumin solution was added to the lecithin dispersion, and then subjected to homogenization under 70°C conditions for 10 minutes. The first homogenized curcumin-lecithin mixed solution was slowly added to purified water, and then subjected to homogenization using a homomixer, to produce nanoparticles (C).

The nanoparticulated curcumin solution was subjected to high pressure homogenization by passing the solution through a microfluidizer at 1,000 bar three times, to prepare a nanocrystallized curcumin complex.

0.3 % by weight of a chitosan (MiraeBiotech, MW≥50,000 dalton) solution (D), which is a cationic hydrogel, at a concentration of 1.5% was added to the nanocrystallized curcumin complex solution, to perform first coating to modify the surface. Then, 0.3 % by weight of a sodium alginate (MSC, MW≥100,000 dalton) solution (E), which is an anionic hydrogel, at a concentration of 0.015% was added thereto to perform second coating to modify the surface. Accordingly, curcumin nanoparticles whose outermost surface is coated with chitosan/alginate were prepared.

### Example 2

Curcumin nanoparticles were prepared in the same manner as in Example 1 except that the adding the sodium alginate solution (E), an anionic hydrogel, in Example 1 above was not performed.

### Example 3

Curcumin nanoparticles were prepared in the same manner as in Example 1 except that the adding the chitosan solution (D), a cationic hydrogel, and the sodium alginate solution (E), an anionic hydrogel, in Example 1 above was not performed.

### Example 4

Curcumin nanoparticles were prepared in the same manner as in Example 1 except that the amount of lecithin was increased to 3.0 % by weight, and the adding the chitosan solution (D), a cationic hydrogel, and the sodium alginate solution (E), an anionic hydrogel, in Example 1 above was not performed.

### Example 5

Curcumin nanoparticles were prepared in the same manner as in Example 1 except that 0.3 % by weight of the chitosan solution (D), a cationic hydrogel, at a concentration of 10% was added, and 0.3 % by weight of 1% sodium alginate solution (E), an anionic hydrogel, was added.

### Example 6

Curcumin nanoparticles were prepared in the same manner as in Example 1 except that the curcumin nanoparticles were prepared in the form of powdered microfine powder by adding 12% by weight of a maltodextrin solution (F) at a concentration of about 33.3% to a carrier solution for powdering and then mixing, and powdering the same by spray drying, after adding the sodium alginate solution (E) in Example 1 above.

**[Table 1]**

| | | Composition of Examples 1 to 6 (unit: % by weight) | | | | | | |
|---|---|---|---|---|---|---|---|---|
| Raw material | | | Ex. 1 | Ex. 2 | Ex. 3 | Ex. 4 | Ex. 5 | Ex. 6 |
| A | Curcumin | | 0.15 | 0.15 | 0.15 | 0.15 | 0.15 | 0.15 |
| | Ethanol | | 7.0 | 7.0 | 7.0 | 7.0 | 7.0 | 7.0 |
| B | Lecithin | | 1.2 | 1.2 | 1.2 | 3.0 | 1.2 | 1.2 |
| | Ethanol | | 5.0 | 5.0 | 5.0 | 5.0 | 5.0 | 5.0 |
| | Purified water | | 17.0 | 17.0 | 17.0 | 17.0 | 17.0 | 17.0 |
| C | Purified water | | up to 100 (an amount that makes the total amount 100 % by weight) | | | | | |
| D | Chitosan | | 0.0045 | 0.0045 | - | - | 0.03 | 0.0045 |
| | Purified water | | 0.2955 | 0.2955 | - | - | 0.27 | 0.2955 |
| E | Sodium alginate | | 0.000045 | - | - | - | 0.003 | 0.000045 |
| | Purified water | | 0.299955 | - | - | - | 0.297 | 0.299955 |
| F | Maltodextrin | | - | - | - | - | - | 4.0 |
| | Purified water | | - | - | - | - | - | 8.0 |

In Table 2, the content of each ingredient of Table 1 is converted into a value relative to 1 part by weight of curcumin.

**[Table 2]**

| | | Composition of Examples 1 to 6 (unit: part by weight) | | | | | | |
|---|---|---|---|---|---|---|---|---|
| Raw material | | | Ex. 1 | Ex. 2 | Ex. 3 | Ex. 4 | Ex. 5 | Ex. 6 |
| A | Curcumin | | 1 | 1 | 1 | 1 | 1 | 1 |
| B | Lecithin | | 8 | 8 | 8 | 20 | 8 | 8 |
| D | Chitosan | | 0.03 | 0.03 | - | - | 0.2 | 0.03 |
| E | Sodium alginate | | 0.0003 | - | - | - | 0.02 | 0.0003 |
| F | Maltodextrin | | - | - | - | - | - | 26.67 |

### Comparative Example

Each ingredient was added according to the composition of Table 3, to prepare coated curcumin nanoparticles.

### Comparative Example 1

0.15 % by weight of curcumin was added to purified water and stirred, to prepare a dispersion.

### Comparative Example 2

Curcumin nanoparticles were prepared in the same manner as in Example 1 except that the adding the chitosan solution (D), a cationic hydrogel, and the sodium alginate solution (E), an anionic hydrogel, in Example 1 above was not performed, and the passing through a microfluidizer was not performed.

### Comparative Example 3

Curcumin nanoparticles were prepared in the same manner as in Example 3 except that the amount of lecithin added in Example 3 above was changed to 0.8 % by weight.

### Comparative Example 4

Curcumin nanoparticles were prepared in the same manner as in Example 2 except that the amount of chitosan, a cationic hydrogel, added in Example 2 above was increased to 0.05 % by weight (i.e., the concentration of the chitosan solution is about 16.7%).

### Comparative Example 5

Curcumin nanoparticles were prepared in the same manner as in Example 1 except that the chitosan solution, a cationic hydrogel, and the sodium alginate solution, an anionic hydrogel, in Example 1 above were added at the same concentration.

**[Table 3]**

| | | Composition of Comparative Examples 1 to 5 (unit: % by weight) | | | | | |
|---|---|---|---|---|---|---|---|
| Raw material | | | Comp. Ex. 1 | Comp. Ex. 2 | Comp. Ex. 3 | Comp. Ex. 4 | Comp. Ex. 5 |
| A | Curcumin | | 0.15 | 0.15 | 0.15 | 0.15 | 0.15 |
| | Ethanol | | - | 7.0 | 7.0 | 7.0 | 7.0 |
| B | Lecithin | | - | 1.2 | 0.8 | 1.2 | 1.2 |
| | Ethanol | | - | 5.0 | 5.0 | 5.0 | 5.0 |
| | Purified water | | - | 17.0 | 17.0 | 17.0 | 17.0 |
| C | Purified water | | up to 100 (an amount that makes the total amount 100 % by weight) | | | | |
| D | Chitosan | | - | - | - | 0.05 | 0.0045 |
| | Purified water | | - | - | - | 0.25 | 0.2955 |
| E | Sodium alginate | | - | - | - | - | 0.0045 |
| | Purified water | | - | - | - | - | 0.2955 |
| Whether to pass through a microfluidizer | | | X | X | O | O | O |

In Table 4, the content of each ingredient of Table 3 is converted into a value relative to 1 part by weight of curcumin.

**[Table 4]**

| | | Composition of Comparative Examples 1 to 5 (unit: part by weight) | | | | | |
|---|---|---|---|---|---|---|---|
| Raw material | | | Comp. Ex. 1 | Comp. Ex. 2 | Comp. Ex. 3 | Comp. Ex. 4 | Comp. Ex. 5 |
| A | Curcumin | | 1 | 1 | 1 | 1 | 1 |
| B | Lecithin | | - | 8 | 5.33 | 8 | 8 |
| D | Chitosan | | - | - | - | 0.33 | 0.03 |
| E | Sodium alginate | | - | - | - | - | 0.03 |

### Experimental Example 1: Comparison of Formulation Stability between Examples and Comparative Examples

Immediately after preparation of Examples 1 to 6 and Comparative Examples 1 to 5, formulation stability was observed with the naked eye. In Examples 1 to 6 which make carriers according to the optimal composition, it was confirmed that immediately after preparation, insoluble curcumin was prepared in a dispersed solution without deposition or precipitation, and maintained the stable form without deposition or precipitation, or layer separation even over time.

In Comparative Example 1 in which insoluble curcumin was dispersed in purified water, it was confirmed that particles were deposited immediately after preparation.

In Comparative Example 2 in which the passing through a microfluidizer was not performed, precipitation was observed from 4 hours, and deposition occurred after 24 hours.

In Comparative Example 3 in which the content of lecithin was reduced, as the amount of lecithin to encapsulate curcumin was not enough, curcumin was deposited.

In Comparative Examples 4 and 5 in which the concentrations of the cationic hydrogel (chitosan) and the anionic hydrogel (sodium alginate) were increased, precipitation and aggregation occurred, and deposition and layer separation occurred due to the gelling in the solution caused by the increased hydrogels.

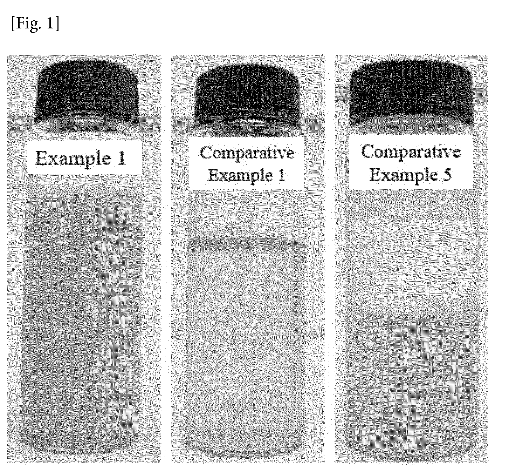

Fig. 1 shows the result of images on the stability of curcumin of Example 1 and Comparative Examples 1 and 5.

**[Table 5]**

| Time | Ex.1 | Ex.2 | Ex.3 | Ex.4 | Ex.5 | Comp. Ex. 1 | Comp. Ex. 2 | Comp. Ex. 3 | Comp. Ex. 4 | Comp. Ex. 5 |
|---|---|---|---|---|---|---|---|---|---|---|
| immediately after preparation | stable | stable | stable | stable | stable | deposited | stable | deposited | stable | aggregated |
| 1 hour | stable | stable | stable | stable | stable | deposited | stable | deposited | precipitated | aggregated |
| 2 hours | stable | stable | stable | stable | stable | deposited | stable | deposited | deposited | aggregated |
| 4 hours | stable | stable | stable | stable | stable | deposited | precipitated | deposited | deposited | layer separated |
| 24 hours | stable | stable | stable | stable | stable | deposited | deposited | deposited | deposited | layer separated |
| 48 hours | stable | stable | stable | stable | stable | deposited | deposited | deposited | deposited | layer |
| | | | | | | | | | | separated |

### Experimental Example 2: Measurement of Particle Size and Particle Formation of Curcumin Nanoparticle Complex

In order to measure the average particle sizes of Examples 1 to 5 in which formulation stability was secured through Experimental Example 1, and Comparative Examples 1 and 2, the particle sizes were measured by a nanoparticle analyzer (Zetasizer, Malvern). In order to check the particle shapes, images were taken by FE-SEM (S-4200, Hitachi).

In order to measure the average particle size, each sample was diluted in a predetermined ratio to set the concentration, to perform the experiment at the same count rate.

The particle size of Example 1 which is coated with chitosan and alginate was measured as 368.2±61.2 nm, the particle size of Example 2 which is coated with chitosan was measured as 340.9±47.88 nm, and the particle size of Example 3 which is uncoated was measured as 321.5±35.04 nm. It was confirmed that the sizes of the carriers increase according to the coatings.

As to zeta potentials, Example 1 was measured as -47.07±0.6255 mV, Example 2 was measured as -40.44±1.1 mV, and Example 3 was measured as -69.27±1.295 mV. It was confirmed that the zeta potentials change each time the particles are coated with the cationic hydrogel and the anionic hydrogel.

It was confirmed that as compared with Examples 1 to 3, in Example 5 in which the contents of the cationic hydrogel and the anionic hydrogel increased (i.e., the concentration of each solution increased), the particle size increased, and a higher poly-dispersity index was measured, which means that the uniformity of particles is relatively low, but no precipitation or deposition occurred, which means that the particles can be used as a carrier.

It was confirmed that in Comparative Example 1 in which curcumin was dispersed in purified water, the particle size was measured as 1658.66±54.90 nm in micro-size. As compared with Example 3, in Comparative Example 2 in which the passing through a microfluidizer was not performed, the particle size was measured as 571.4±107.2 nm, which is a non-uniform particle size of 500 nm or more.

**[Table 6]**

| | Ex. 1 | Ex. 2 | Ex. 3 | Ex. 4 | Ex. 5 | Comp. Ex. 1 | Comp. Ex. 2 |
|---|---|---|---|---|---|---|---|
| Particle size (nm) | 368.2± 61.2 | 340.9± 47.88 | 321.5± 35.04 | 317.86± 37.19 | 817.7± 245.3 | 1658.66± 54.90 | 571.4± 107.2 |
| Poly-dispersity index | 0.1121± 0.05771 | 0.2211± 0.0276 | 0.2573± 0.0899 | 0.2698± 0.0605 | 0.8205± 0.1794 | 0.778± 0.059 | 0.6549± 0.451 |
| Zeta potential (mV) | -47.07± 0.6255 | -40.44± 1.1 | -69.27± 1.295 | -70.53± 1.324 | -23.93± 0.9369 | -31.46± 1.847 | -53.61± 0.5313 |

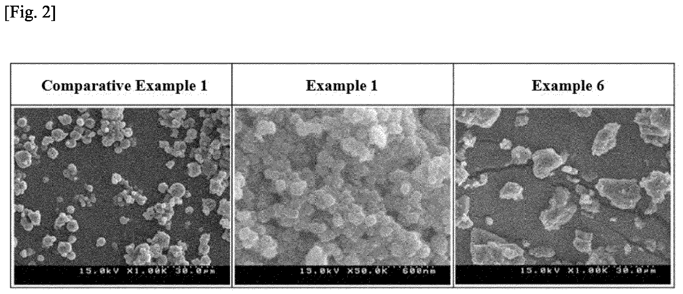

Fig. 2 shows FE-SEM images. In Comparative Example 1, amorphous particles were observed. In Example 1, nanocomposites of uniform particles were observed. In Example 6 in which spray drying was performed, microcomposites of spherical uniform particles were observed.

### Experimental Example 3: Encapsulation Efficiency of Curcumin Nanoparticle Complex

In order to confirm the encapsulation efficiency of curcumin which is the active ingredient encapsulated in a drug carrier for Examples 1 to 3 which were confirmed through Experimental Example 2 that the particle sizes are uniform, the encapsulation efficiency was measured by high performance liquid chromatography (Shimadzu).

A standard solution at each concentration point for measuring the encapsulation efficiency of curcumin was prepared at a concentration of 1 mg/mL using ethyl acetate. The prepared solution at each concentration was diluted with ethyl acetate to be 0.2 to 100 µg/mL, to prepare standard solutions for calibration curves. The prepared calibration curves were prepared such that the ethyl acetate standard solutions have high linearity of 0.99999 within the concentration range.

1 mL of the solutions prepared in Examples 1 to 3 were put in glass test tubes, and 20 mL of ethyl acetate was added thereto and dispersed with a vortex mixer for 1 minute, followed by shaking with an ultrasonic shaker for 1 hour, to extract curcumin in organic solvent phases. The organic solvent phases were collected and separated in a centrifugation at 11,000 g at 4°C for 20 minutes, to use the supernatants as total concentration analysis samples.

1 mL of the solutions prepared in Examples 1 to 3 were collected and put in Amicon^{®} Ultra-4 centrifugal filter, and filtered in a centrifugation at 7,500 g at 25°C for 30 minutes. The recovered lower solutions were used as non-encapsulated concentration measurement samples.

For the LC-UV/RFD used herein, the UV-Vis detector SPD-40 of Shimadzu and the fluorescence detector RF-20A were used, to establish analysis conditions.

The mobile phase A is ACN, and the mobile phase B is 0.1% formic acid solution.

The initial mobile phase A was flown in a volume ratio of 20% at a flow rate of 1 mL/min in total, with a linear gradient for the concentration gradient of the mobile phase such that the ratio of the mobile phase A becomes 79.6% at 20 minutes. Then, the samples were washed with ACN and methanol in a volume ratio of 50:50 for 10 minutes in order to prevent cross-contamination between the samples, followed by stabilization under the initial mobile phase conditions for 10 minutes. Thereafter, the samples were measured.

For the column, CAPCELL PAK C18 UG 120 (4.6 X 250 mm) 5µm column was used, and the temperature was maintained at 35°C.

The UV-Vis detector was analyzed at a wavelength of 430 nm, and the fluorescence detector was analyzed under Em 420 nm, Ex 550 nm conditions.

**[Table 7]**

| Encapsulation efficiency of Examples 1 to 3 | | | |
|---|---|---|---|
| | Ex. 1 | Ex. 2 | Ex. 3 |
| Encapsulation efficiency (%) | 99.911 | 99.893 | 99.811 |

The encapsulation efficiency of the nanocrystallized curcumin of Example 3 was calculated as about 99.811% with respect to the total curcumin concentration in the sample filtered with 3k dalton. The encapsulation efficiency of Example 2 and the encapsulation efficiency of Example 1 were measured as 99.893% and 99.911%, respectively.

### Experimental Example 4: Efficiency of LPS-induced Nitric Oxide (NO) Inhibition of Curcumin Nanoparticle Complex

The efficiency of LPS-induced nitric oxide (NO) inhibition of the nanocrystallized curcumin complex was measured using Examples 1 and 2 and Comparative Example 1.

RAW 264.7 and MOLT-4, which are a macrophage cell line and an immune cell line, respectively, were purchased from American Type Culture Collection (ATCC, Rockville, MD, USA), and cultured in Dulbeccos Modified Eagles Medium (DMEM; GIBCO Inc.), RPMI-1640 supplemented with 10% fetal bovine serum (FBS), penicillin (100 units/mL) and streptomycin (100 g/mL). These cells were cultured under 5% CO₂ conditions at 37°C, and subcultured every three days.

RAW 264.7 cells were plated in a 96 well plate at 1 × 10⁶ cells/mL and cultured for 24 hours. Then, the cells were treated with the samples and with LPS (1 µg/mL) to induce an inflammatory response for 24 hours. Thereafter, the cell supernatant and the same amount of the Griess reagent (N-1-naphthylenthylenediamine, sulfanilamide) were mixed and reacted in a 96 well plate for 10 minutes, to measure NO production at an absorbance of 540 nm using a spectrophotometer.

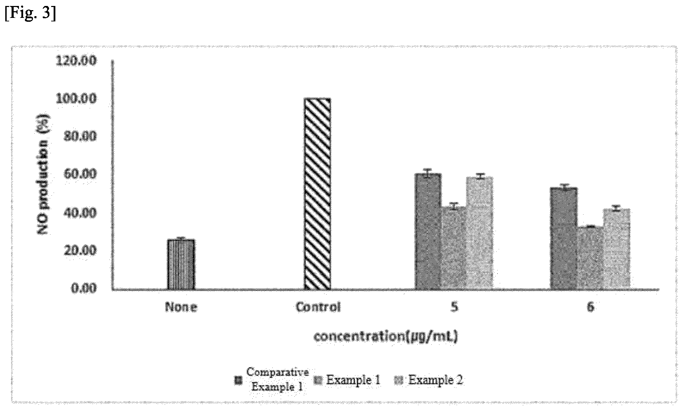

The result was shown in Fig. 3. As a result of measurement of NO production, it was confirmed that as compared with the LPS group, control, in Examples 1 and 2, NO production was reduced in a concentration-dependent manner.

### Experimental Example 5: Measurement of TNF-α Expression Level of Curcumin Nanoparticle Complex (Western blot)

RAW 264.7 cells were plated in a 6 well plate at 1 × 10⁶ cells/ml and cultured for 24 hours. Then, the cells were treated with 6 µg/ml of Comparative Example 1 and Examples 1 and 2 and with LPS (1 µg/ml), inflammatory agent, to induce inflammation for 24 hours. Untreated cells were used as normal control (None; N), and cells treated with LPS to induce inflammation for 24 hours were used as control (Control; C).

After the supernatants were removed 24 hours later and the cells were washed twice with PBS, the RIPA buffer was added thereto in an amount of 200 µl/well to homogenize the cells. The homogenized cells were centrifuged at 12,000 rpm at 4°C for 10 minutes to collect the supernatants. The concentrations of proteins were quantified based on bovine serum albumin (BSA).

The quantified protein samples were electrophoresed using 10% spolyacrylamide gel and transferred to polyvinylidene fluoride membranes. The membranes were then blocked in the blocking buffer for 1 hour using 2% skim milk, and washed using TBST (TBS + 0.1% Tween 20). Then, the TNF-α primary antibody was incubated at 4°C for 24 hours. Thereafter, the secondary antibody was added thereto and reacted at room temperature for 1 hour, followed by development in the darkroom using ECL (Amersham) by X-ray film luminescence. The TNF-α production was quantified using Western blot.

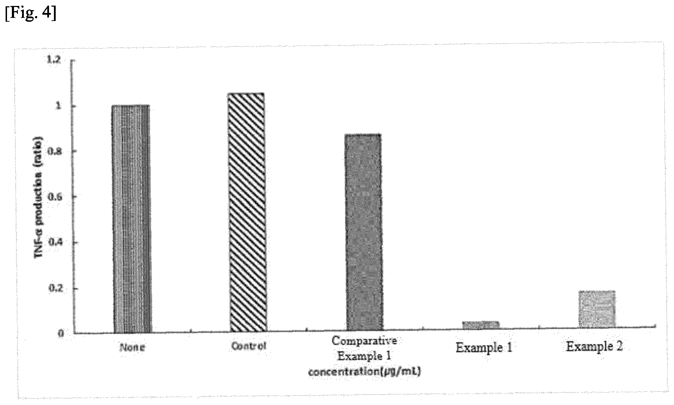

The result was shown in Fig. 4. As a result of experiment, it could be understood that in Examples 1 and 2, the rate of an increase in TNF-α production was reduced.

### Experimental Example 6: MOLT-4 Cells Proliferation Capacity of Curcumin Nanoparticle Complex

MOLT-4 cells were plated in a 6 well plate at 5 × 10⁵ cells/mL and treated with Examples 1 and 2 and Comparative Example 1, nanocrystallized curcumin complexes, at each concentration (5 to 6 µg/ml), and then cultured for 24 hours. Untreated cells were used as normal control (None; N). Cells to which Concanvalin A (1 µg/ml) for T cell proliferation and LPS (1 µg/ml) for B cell proliferation were added were used as positive control (Control). The cells were cultured under 5% CO₂ conditions at 37°C for about 72 hours, to measure T cell proliferation.

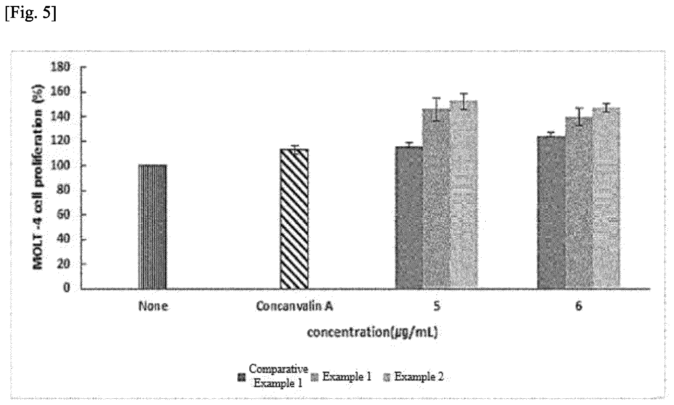

The result was shown in Fig. 5. As a result of measurement of T cell (i.e., MOLT-4 cell) proliferation, when assuming that the T cell proliferation of normal control is 100%, the T cell proliferation was measured as 115.92% when the concentration of curcumin is 5 µg/ml, which is higher than 112.79%, the T cell proliferation of positive control. The T cell proliferations of Examples 1 and 2, nanocrystallized curcumin microparticle complexes, were measured as 145.88% and 152.23%, respectively. It could be confirmed that in Examples 1 and 2 which were prepared in nanocrystallized curcumin microparticle complexes, the amount of MOLT-4 cells, T cells, was increased more than in Comparative Example 1 and the positive control Concanvalin A.

### Experimental Example 7: in vitro Release Testing of Nanocrystallized Curcumin Microparticle Complex

In order to confirm the amount of release of Example 6 and Comparative Example 1 of curcumin under artificial gastro fluid and artificial intestinal fluid conditions, *in vitro* release testing was carried out under each pH condition.

The release testing was carried out using a dialysis bag (Thermo scientific, 3k dalton molecular cut off). 4 mL of each of the solutions of Example 6 and Comparative Example 1 was added to the upper part of the dialysis bag, and 44 mL of each of the artificial gastro fluid and artificial intestinal fluid was added to the lower part thereof.

Stirring with a constant temperature stirrer at 200 RPM at 36.5°C, 4 mL was collected at 1, 2, 4, 8, 24 and 48 hours, and the same amounts of the artificial gastro fluid and artificial intestinal fluid were filled therein.

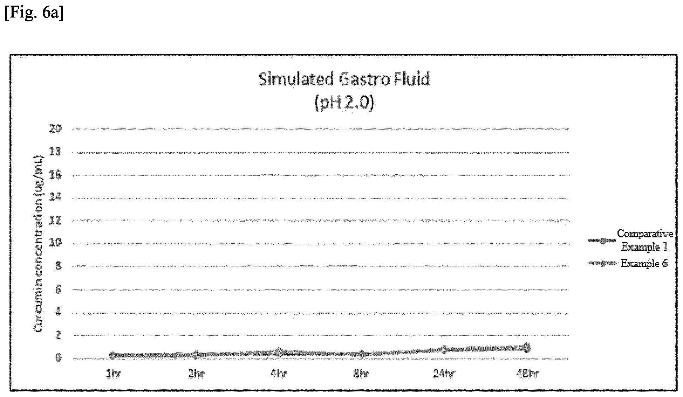

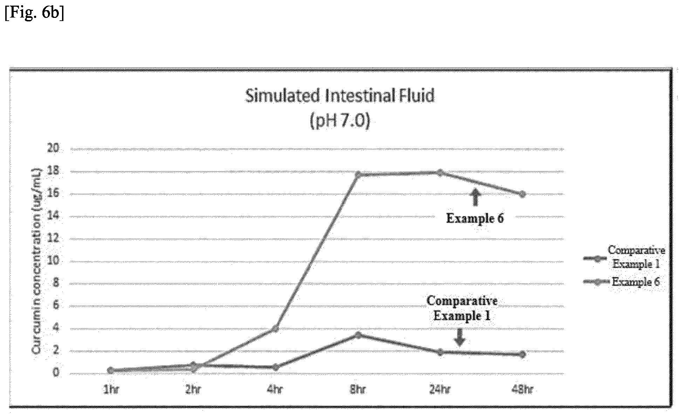

The result was shown in Fig. 6. Under the artificial gastro fluid conditions, neither Example 6 nor Comparative Example 1 was released (Fig. 6a; the graphs of Example 6 and Comparative Example 1 almost overlap with each other). Under the artificial intestinal fluid conditions, it was confirmed that a much larger amount of Example 6 was released than Comparative Example 1 after 4 hours, and the released amount was increased up to four times or more (Fig. 6b).

### Experimental Example 8: Evaluation of Intestinal Absorption of Nanocrystallized Curcumin Microparticle Complex through In Vivo Animal Testing

In order to evaluate the intestinal absorption of Example 6 and Comparative Example 1, *in vivo* animal testing was carried out through SD rats.

*In vivo* animal testing was carried out after obtaining prior approval from the Institutional Animal Care and Use Committee (Examination No. IACUC-UR-2022-01).

8-week-old male SD rats were fasted 12 hours before oral administration, and Example 6 and Comparative Example 1 were orally administered in an amount of 200 mg/kg based on curcumin.

The blood samples were collected via retro-orbital bleeding, at 30 minutes, and 1, 2, 4, 8 and 24 hours. Right after blood collection, the supernatant plasmas were separated by centrifugation at 3000 rpm for 5 minutes and stored at -80°C until measurement.

For the standard samples used in the testing, a highly pure curcumin reagent was purchased and used.

The standard samples were prepared using acetonitrile (ACN) at a concentration of 0.5 mg/mL and stored at -80°C.

The standard solution at each concentration point for measuring the blood curcumin concentration was prepared by diluting the prepared standard reagent solution with HPLC grade acetonitrile (ACN) solvent.

The prepared concentration point in a range of 5 to 500 ng/mL was used as standard solutions for calibration curve.

The prepared calibration curve was prepared as a calibration curve with high reliability of 0.9907 within the concentration range.

200 µL of each of the blood samples collected from the rats was taken and put into a glass tube, and 25 µL of 2.8% acetic acid solution was added thereto, followed by stirring with a vortex mixer for 20 seconds.

1.2 mL of ethyl acetate was added to the stirred solutions, followed by mixing with a vortex mixer for 1 minute and ultrasonic treatment for 10 minutes.

The mixed solutions were separated by centrifugation at 3000 rpm for 10 minutes and 1 mL of the primary organic phases were transferred to clean glass tubes.

The transferred organic solutions were evaporated in a nitrogen concentrator at 40°C for 25 minutes to completely volatilize the organic solutions.

100 µL of ACN was put into the glass tubes in which the organic solutions were completely volatilized, followed by stirring with a vortex mixer for 20 seconds to be completely dissolved, thereby obtaining measurement samples.

For LC-MS/MS used herein, LCMS-8050 was used to establish LC-MS/MS conditions.

The mobile phase A is ACN, and the mobile phase B is 2 mM ammonium acetate and 0.1% formic acid solution. The mobile phase A was flown in a volume ratio of 95% at a flow rate of 0.2 mL/min in total using a double pump, with a linear gradient for the concentration gradient of the mobile phase such that the rate of the mobile phase A is retained to be 95% until 0.5 minutes, and then the rate of the mobile phase A is 5% at 1.5 minutes, the mobile phase A 5% at 5 minutes and the mobile phase A 95% at 6 minutes. The final composition was maintained for 10 minutes.

For the column, Atlantis^{™} T3 3µm (2.1 × 100 mm) column was used, and the temperature was maintained at 30°C.

The molecular weights of DHA designated by multiple reaction monitoring (MRM) of the LC-MS detector was analyzed with m/z 367.05>134.00.

The LC-MS detector was operated under the following conditions: interface electrospray ionization (ESI); inert gas argon; nebulizing gas flow 3 L/min; drying gas flow 10 L/min; heating gas flow 10 L/min; interface temperature 300°C; DL temperature 250°C; heating block temperature 400°C; collision-induced dissociation (CID) gas 17 kPa; and interface voltage 2.24 kV.

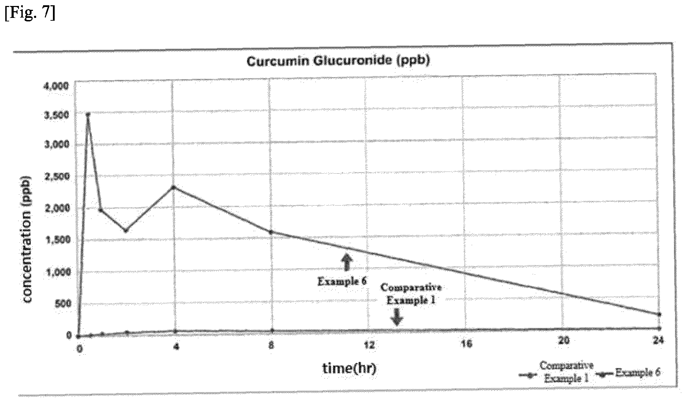

The results were shown in Fig. 7 and Table 8. Example 6 has a higher Cmax value than Comparative Example 1, and has about 43 times higher AUC, area under the plasma level-time curve, which is the index of bioavailability.

**[Table 8]**

| | Cmax (ng/mL) | AUC 24hr (ng*min/mL) |
|---|---|---|
| Ex. 6 | 3471.36±1852.00 | 30242.75 |
| Comp. Ex. 1 | 57.37±38.64 | 690.85 |

## Claims

1. A curcumin nanoparticle complex comprising:
curcumin; and
6 parts by weight or more of lecithin relative to 1 part by weight of curcumin,
wherein the curcumin nanoparticle complex can further comprise:
a first coating layer comprising 0 to 0.3 parts by weight of chitosan relative to 1 part by weight of curcumin; or
a second coating layer comprising 0 to 0.025 parts by weight of sodium alginate relative to 1 part by weight of curcumin,
wherein the second coating layer is comprised only when the first coating layer is comprised.

2. The curcumin nanoparticle complex of claim 1, comprising 8 parts by weight or more of lecithin relative to 1 part by weight of curcumin.

3. The curcumin nanoparticle complex of claim 1, comprising 8 to 20 parts by weight of lecithin relative to 1 part by weight of curcumin.

4. The curcumin nanoparticle complex of claim 1, wherein the first coating layer comprises 0.03 to 0.2 parts by weight of chitosan relative to 1 part by weight of curcumin.

5. The curcumin nanoparticle complex of claim 1, wherein the second coating layer comprises 0.0003 to 0.02 parts by weight of sodium alginate relative to 1 part by weight of curcumin.

6. The curcumin nanoparticle complex of claim 1, comprising:
curcumin; and
8 to 20 parts by weight of lecithin relative to 1 part by weight of curcumin.

7. The curcumin nanoparticle complex of claim 1, comprising:
curcumin;
8 parts by weight of lecithin relative to 1 part by weight of curcumin; and
a first coating layer comprising 0.03 parts by weight of chitosan relative to 1 part by weight of curcumin.

8. The curcumin nanoparticle complex of claim 1, comprising:
curcumin;
8 parts by weight of lecithin relative to 1 part by weight of curcumin;
a first coating layer comprising 0.03 parts by weight of chitosan relative to 1 part by weight of curcumin; and
a second coating layer comprising 0.0003 parts by weight of sodium alginate relative to 1 part by weight of curcumin.

9. The curcumin nanoparticle complex of claim 1, comprising:
curcumin;
8 parts by weight of lecithin relative to 1 part by weight of curcumin;
a first coating layer comprising 0.2 parts by weight of chitosan relative to 1 part by weight of curcumin; and
a second coating layer comprising 0.02 parts by weight of sodium alginate relative to 1 part by weight of curcumin.

10. A microfine powder powdered from a curcumin nanoparticle complex, comprising:
the curcumin nanoparticle complex of any one of claims 1 to 9; and
at least one water soluble polymer selected from the group consisting of maltodextrin, dextrin and starch.

11. The microfine powder powdered from a curcumin nanoparticle complex of claim 10, wherein the water soluble polymer is comprised in an amount of 20 to 30 parts by weight relative to 1 part by weight of curcumin.

12. A microfine powder powdered from a curcumin nanoparticle complex, comprising:
the curcumin nanoparticle complex of claim 8; and
20 to 30 parts by weight of maltodextrin relative to 1 part by weight of curcumin.

13. A composition comprising the curcumin nanoparticle complex of any one of claims 1 to 9.

14. A method for preparing the curcumin nanoparticle complex of claim 1, comprising:
(a) dissolving curcumin in an organic solvent to prepare a curcumin solution;
(b) dissolving lecithin in an organic solvent, water or a mixed solvent of the two to prepare a lecithin solution or dispersion;
(c) mixing the curcumin solution of step (a) with the lecithin solution or dispersion of step (b) to prepare a curcumin-lecithin mixed solution;
(d) mixing the curcumin-lecithin mixed solution with water to form an emulsion; and
(e) high-pressure homogenizing the emulsion to prepare a curcumin nanoparticle complex,
and optionally comprising:
(f) performing first coating on the curcumin nanoparticle complex with a first coating agent comprising chitosan; or
(g) performing second coating on the first coated curcumin nanoparticle complex with a second coating agent comprising sodium alginate.

15. The method of claim 14, wherein in step (f), the first coating agent comprising chitosan is a chitosan solution having a concentration of 1.5%(w/w) or more.

16. The method of claim 14, wherein in step (g), the second coating agent comprising sodium alginate is a sodium alginate solution having a concentration of 0.015%(w/w) or more.

17. A method for preparing the microfine powder of claim 10, comprising:
preparing a curcumin nanoparticle complex according to claim 14;
(g) adding at least one water soluble polymer selected from the group consisting of maltodextrin, dextrin and starch; and
(h) performing powdering through spray drying or freeze drying.
